# EUROPÄISCHE PATENTSCHRIFT

(11) **EP 0 519 246 B1**
(45) Veröffentlichungstag und Bekanntmachung des Hinweises auf die Patenterteilung: **14.12.1994**
(21) Anmeldenummer: 92109044.5
(22) Anmeldetag: 29.05.1992
(51) Int. Cl.: C07C 303/22, C07C 309/51

(54) **Verfahren zur Herstellung von N,N'-bis-[7-(4-hydroxy-2-sulfo)naphthyl]-harnstoff**
Process for the preparation of N,N'-bis-[7-(4-hydroxy-2-sulfo)naphthyl]-urea
Procédé pour la préparation de N,N'-bis-[7-(4-hydroxy-2-sulfo)naphthyl]-urée

(30) Priorität: 20.06.1991 DE 4120366
(43) Veröffentlichungstag der Anmeldung: 23.12.1992
(73) Patentinhaber: BASF Aktiengesellschaft, 67063 Ludwigshafen (DE)
(72) Erfinder: Hahn, Erwin, Dr., W-6900 Heidelberg (DE); Mayer, Udo, Dr., W-6710 Frankenthal (DE); Raulfs, Friedrich-Wilhelm, Dr., W-6703 Limburgerhof (DE); Schloesser, Ulrike, Dr., W-6800 Mannheim 81 (DE)

(56) Entgegenhaltungen:
- DE-C- 116 200
- US-A- 4 591 604
- CHEMICAL ABSTRACTS, Bd. 68, Nr. 16, 15. April 1968, Columbus, Ohio, US; I. A. ABROMOV et al, "J-Acid urea", Zusammenfassung Nr. 70180m, Seite 6791, Spalte 2
- CHEMICAL ABSTRACTS, Bd. 86, Nr. 8, 21. Februar 1977, Columbus, Ohio, US; J. KROUPA et al, "Improvement of the production of phosgenated dyes", Zusammenfassung Nr. 44738b, Seite 53, Spalte 1

## Beschreibung

Die vorliegende Erfindung betrifft ein neues Verfahren zur Herstellung von I-Säure-harnstoff aus I-Säure (7-Amino-4-hydroxynaphthalin-2-sulfonsäure) und Harnstoff.

I-Säure-harnstoff wird üblicherweise durch Reaktion von I-Säure mit Phosgen hergestellt (s. z.B. DE-Patent Nr. 116 200 oder PL-Patent Nr. 125 691).

Aufgabe der vorliegenden Erfindung war es, ein neues Verfahren zur Herstellung von I-Säure-harnstoff bereitzustellen, das die Verwendung von Phosgen als Reagens vermeidet und mittels dessen das Zielprodukt in einfacher Weise und hoher Ausbeute und Reinheit erhalten wird.

Es wurde nun gefunden, daß die Herstellung von I-Säure-harnstoff der Formel 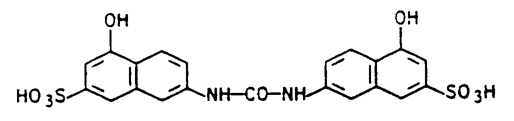
vorteilhaft gelingt, wenn man 7-Amino-4-hydroxynaphthalin-2-sulfonsäure der Formel 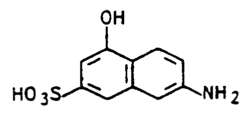
mit Harnstoff bei einer Temperatur von 100 bis 140°C und einem Druck von Normaldruck bis 10 bar in einem inerten Lösungsmittel partiell umsetzt, nicht umgesetzte I-Säure im sauren Milieu fällt und abtrennt und aus der resultierenden neutralisierten Lösung I-Säure-harnstoff gegebenenfalls aussalzt.

Geeignete Lösungsmittel, die im erfindunsgemäßes Verfahren zur Anwendung kommen können, sind z.B. Wasser, C₁-C₄-Alkanole, wie Methanol, Ethanol, Propanol, Isopropanol, Butanol, Isobutanol oder sec-Butanol, Gemische dieser Lösungsmittel oder verdünnte wäßrige Mineralsäuren, z.B. 1 bis 5 gew.-%ige Salzsäure, 1 bis 5 gew.-%ige Bromwasserstoffsäure oder 1 bis 5 gew.-%ige Schwefelsäure.

Die Verwendung von Wasser als Lösungsmittel ist hervorzuheben.

Das erfindungsgemäße Verfahren wird zweckmäßig so durchgeführt, daß man in einer geeigneten Apparatur, z.B. in einem Druckkessel, Harnstoff, I-Säure und Lösungsmittel vorlegt. Dabei beträgt das Molverhältnis Harnstoff zu I-Säure in der Regel 1 : 2 bis 4 : 1, vorzugsweise ca. 1 : 2. Bezogen auf das Gesamtgewicht von I-Säure und Harnstoff werden in allgemeinen 30 bis 80 Gew.-%, vorzugsweise 50 bis 70 Gew.-% an Lösungsmittel verwendet.

Das Gemisch wird dann, in der Regel unter Rühren, auf eine Temperatur von 100 bis 140°C, vorzugsweise 110 bis 130°C und insbesondere 115 bis 125°C erhitzt. Dabei wird ein Druck von Normaldruck bis 10 bar, vorzugsweise 2 bis 6 bar und insbesondere 3 bis 5 bar eingestellt. Dies kann z.B. durch entsprechendes Einstellen eines überströmventils erfolgen, wobei das bei der Reaktion entstehende Ammoniakgas, Lösungsmitteldampf sowie in geringen Mengen durch Zersetzung von I-Säure-harnstoff entstehendes Kohlendioxid entweichen kann.

Nach einer Dauer von 2 bis 40 Stunden, wobei die obengenannten Parameter eingehalten werden, wird die Reaktion abgebrochen und das Reaktionsgemisch abgekühlt und entspannt.

Zu diesem Zeitpunkt ist das Reaktionsgemisch partiell umgesetzt und es sind in der Regel 10 bis 60 %, vorzugsweise 40 bis 60 %, der theoretischen Menge an I-Säure-harnstoff gebildet worden.

Es kann dann weiteres Lösungsmittel, im allgemeinen 0 bis 30 Gew.-%, bezogen auf das Gesamtgewicht von Harnstoff und I-Säure, zugegeben werden.

Danach wird das Reaktionsgemisch mit wäßriger Mineralsäure, z.B. 30 gew.-%iger Salzsäure, versetzt und ein pH-Wert von 1,5 bis 2,5 eingestellt und auf diese Weise nicht umgesetzte I-Säure gefällt. Sie kann beispielsweise durch Absaugen abgetrennt werden.

Die so abgetrennte I-Säure kann nach dem Waschen mit Wasser und Trocknung im erfindungsgemäßen Verfahren wieder zur Anwendung kommen.

Das den I-Säure-harnstoff enthaltende Filtrat wird dann mit Base, z.B. mit 30 bis 50 gew.-%iger Natron- oder Kalilauge neutralisiert, d.h. es wird auf einen pH-Wert von 5 bis 8, vorzugsweise ca. 6,5 eingestellt und ausgesalzen, was z.B. durch Zugabe von 30 bis 70 Gew.-% Natriumchlorid, bezogen auf das Gesamtgewicht von I-Säure und Harnstoff, erreicht werden kann. Danach wird das Verfahrensprodukt abgesaugt, getrocknet und gegegebenfalls gemahlen.

Mittels des erfindungsgemäßen Verfahrens kann I-Säure-harnstoff auf einfachem Wege in guter Ausbeute und in hoher Reinheit hergestellt werden, wobei die Verwendung von Phosgen als Reaktionspartner vermieden wird.

Die im erfindungsgemäßen Verfahren zunächst anfallende I-Säure-harnstofflösung ist von so hoher Reinheit, daß sie in geeigneten Fällen auch direkt weiterverarbeitet werden kann.

I-Säureharnstoff ist ein wertvolles Zwischenprodukt für die Herstellung von Azofarbstoffen. Er dient dabei als Kupplungskomponente.

Das folgende Beispiel soll die Erfindung näher erläutern.

### Beispiel 1

In einem 4 l Rührkessel wurden 120 g (2 mol) Harnstoff und 956 g (4 mol) 7-Amino-4-hydroxy-2-naphthalinsulfonsäure (I-Säure) in 2000 ml Wasser innerhalb von 2 Stunden auf 120°C erhitzt und bei einem Druck von 3,5 bar 4 Stunden bei dieser Temperatur belassen. Nach dem Abkühlen wurde die resultierende Suspension mit Wasser auf eine Gesamtmenge von 3300 g verdünnt und mit 30 gew.-%iger Salzsäure auf einen pH-Wert von 2 gestellt.

Die ausgefallene I-Säure wurde abgesaugt und mit Wasser gewaschen. Der pH-Wert des resultierenden Filtrats wurde mit 30 gew.-%iger Salzsäure auf 1,5 erniedrigt, wobei nochmals I-Säure ausfiel, die mit der oben erhaltenen Menge vereinigt wurde. Nach dem Trocknen bei 60°C und anschließendem Mahlen resultierten insgesamt 386 g (37 %) I-Säure in Form eines hellgrauen Pulvers.

Das bei der Abtrennung der I-Säure vom Reaktionsgemisch resultierende Filtrat wurde mit 50 gew.-%iger Natronlauge auf einen pH-Wert von 6,5 gestellt und mit Natriumchlorid gesättigt, wobei der I-Säure-harnstoff ausfiel. Nach Absaugen, Trocknen bei 60°C und Mahlen resultierten 600 g (50 %) I-Säure-harnstoff in Form eines hellbraunen Pulvers mit einem Natriumchloridgehalt von 19 Gew.-%.

### Beispiel 2

In einem 4 l Rührkessel wurden 120 g (2 mol) Harnstoff und 956 g (4 mol) 7-Amino-4-hydroxy-2-naphthalinsulfonsäure (I-Säure) in 2000 ml Wasser innerhalb von 2 Stunden auf 120°C erhitzt und bei einem Druck von max. 8 bar 4 Stunden bei dieser Temperatur belassen. Nach Zugabe von 195 g (0,53 mol) 10 gew.-%iger Salzsäure und weiteren 4 Stunden bei 120°C und max. 7 bar erfolgte die Aufarbeitung wie in Beispiel 1 beschrieben.

Insgesamt resultierten 565 g (48 %) I-Säure, die noch 13 Gew.-% an I-Säure-harnstoff enthielt, und 454 g (37 %) I-Säure-harnstoff mit einem Natriumchloridgehalt von 19 Gew.-%.

### Beispiel 3

In einem 4 l Rührkessel wurden 60 g (1 mol) Harnstoff und 256 g (1 mol) 7-Amino-4-hydroxy-2-naphthalinsulfonsäure (I-Säure) in 500 ml Wasser 16 Stunden unter Rückfluß erhitzt. Nach Zugabe von 190 ml 10 gew.-%iger Salzsäure, 256 g (1 mol) I-Säure und 250 ml Wasser wurde weitere 17 Stunden unter Rückfluß erhitzt.

Die Aufarbeitung erfolgte wie unter Beispiel 1 beschrieben.

Insgesamt resultierten 215 g (35 %) I-Säure, die noch 14 Gew.-% an I-Säure-harnstoff enthielt, und 331 g (50 %) I-Säure-harnstoff mit einem Natriumchloridgehalt von 22 Gew.-%.

## Patentansprüche

1. Verfahren zur Herstellung von I-Säure-harnstoff der Formel 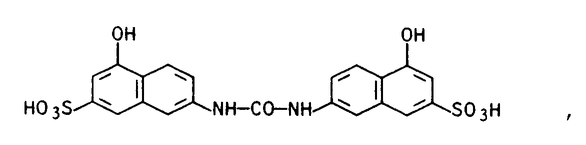 dadurch gekennzeichnet, daß man 7-Amino-4-hydroxynaphthalin-2-sulfonsäure (I-Säure) der Formel 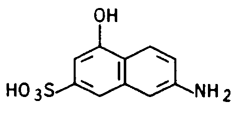 mit Harnstoff bei einer Temperatur von 100 bis 140°C und einem Druck von Normaldruck bis 10 bar in einem inerten Lösungsmittel partiell umsetzt, nicht umgesetzte I-Säure im sauren Medium fällt und abtrennt und aus der resultierenden neutralisierten Lösung I-Säure-harnstoff gegebenenfalls aussalzt.

2. Verfahren nach Anspruch 1, dadurch gekennzeichnet, daß man als inertes Lösungsmittel Wasser, C₁-C₄-Alkanole, Gemische dieser Lösungsmittel, oder verdünnte wäßrige Mineralsäuren verwendet.

3. Verfahren nach Anspruch 1, dadurch gekennzeichnet, daß man die Umsetzung bei einer Temperatur von 110 bis 130 vornimmt.

4. Verfahren nach Anspruch 1, dadurch gekennzeichnet, daß man die Umsetzung bei einem Druck von 2 bis 6 bar vornimmt.

## Claims

1. A process for preparing J-acid urea of the formula 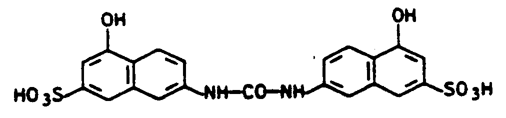 which comprises reacting 7-amino-4-hydroxynaphthalene-2-sulfonic acid (J-acid) of the formula 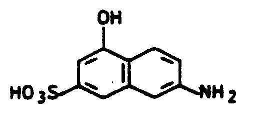 with urea at from 100 to 140°C under a pressure ranging from atmospheric pressure to 10 bar in an inert solvent to a partial extent, precipitating unconverted J-acid in an acidic medium and separating it off, and, if desired, isolating J-acid urea from the resulting neutralized solution by salting out.

2. A process as claimed in claim 1, wherein the inert solvent used is water, a C₁-C₄-alkanol, a mixture thereof, or a dilute aqueous mineral acid.

3. A process as claimed in claim 1, wherein the reaction is carried out at a temperature from 110 to 130.

4. A process as claimed in claim 1, wherein the reaction is carried out under a pressure of from 2 to 6 bar.

## Revendications

1. Procédé de préparation d'acide I-urée de formule 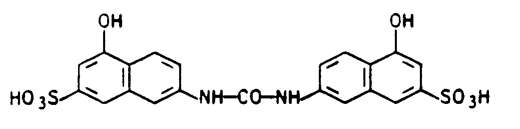 caractérisé en ce qu'on fait réagir partiellement de l'acide 7-amino-4-hydroxynaphtaléne-2-sulfonique (acide I) de formule 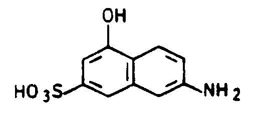 avec de l'urée a une température de 100 a 140°C et sous une pression comprise entre la pression normale et 10 bar, dans un solvant inerte, on précipite en milieu acide et on sépare l'acide I n'ayant pas réagi et on relargue éventuellement l'acide I-urée a partir de la solution neutralisée résultante.

2. Procédé selon la revendication 1, caractérisé en ce qu'on utilise, comme solvant inerte, de l'eau, des alcanols en C₁-C₄, des mélanges de ces solvants ou des acides minéraux aqueux dilués.

3. Procédé selon la revendication 1, caractérisé en ce qu'on conduit la réaction à une température de 110 à 130°C.

4. Procédé selon la revendication 1, caractérisé en ce qu'on conduit la réaction sous une pression de 2 à 6 bar.
